## Europäisches Patentamt
## European Patent Office
## Office européen des brevets

(11) Veröffentlichungsnummer: **0 043 922**
**B1**

(12) **EUROPÄISCHE PATENTSCHRIFT**

(45) Veröffentlichungstag der Patentschrift:
19.09.84

(21) Anmeldenummer: 81104591.3

(22) Anmeldetag: 15.06.81

(51) Int. Cl.³: **C 07 D 233/92**

(54) Verfahren zur Herstellung von 1.2-Dimethyl-5-nitroimidazol.

(30) Priorität: 16.07.80 DE 3026846

(43) Veröffentlichungstag der Anmeldung:
20.01.82 Patentblatt 82/3

(45) Bekanntmachung des Hinweises auf die Patenterteilung:
19.09.84 Patentblatt 84/38

(84) Benannte Vertragsstaaten:
**DE FR GB IT NL**

(56) Entgegenhaltungen:
**DE - A - 2 414 280**
**DE - C - 1 084 729**
**FR - A - 2 263 241**

(73) Patentinhaber: **BASF Aktiengesellschaft,
Carl-Bosch-Strasse 38, D-6700 Ludwigshafen (DE)**

(72) Erfinder: **Hauffe, Werner, Dr., Dackenheimer Strasse 3,
D-6700 Ludwigshafen (DE)**
Erfinder: **Magnussen, Peter, Dr.,
Professor-Dillinger-Weg 25, D-6702 Bad Duerkheim (DE)**
Erfinder: **Dockner, Toni, Dr., Grossgasse 6,
D-6701 Meckenheim (DE)**

## Beschreibung

Die Erfindung betrifft ein Verfahren zur Herstellung von 1,2-Dimethyl-5-nitroimidazol durch Umsetzung von 2-Methyl-4(5)-nitroimidazol mit Dimethylsulfat in Ameisensäure bei vermindertem Druck.

1-Alkyl-5-nitroimidazole lassen sich z. B. nach einem in der DE-OS 2 414 280 beschriebenen Verfahren durch Umsetzung von 5-Nitroimidazolen mit Alkylierungsmitteln in aliphatischen Carbonsäuren herstellen. So erhält man nach diesem Verfahren 1,2-Dimethyl-5-nitroimidazol, wenn man 2-Methyl-4(5)-nitroimidazol (Menidazol) in Ameisensäure bei Temperaturen von 20 bis 110°C und bei Normal- oder Überdruck mit Dimethylsulfat behandelt.

Bei diesem bekannten Verfahren werden nicht unbeträchtliche Mengen an Ameisensäure und Dimethylsulfat infolge Bildung von Methylformiat verbraucht. Es war deshalb nach einem Verfahren zu suchen, das es gestattet, 1,2-Dimethyl-5-nitroimidazol auf wirtschaftlichere Weise und mit besseren Ergebnissen hinsichtlich Ausbeute und Reinheit herzustellen.

Es wurde nun gefunden, daß man bei der Herstellung von 1,2-Dimethyl-5-nitroimidazol durch Umsetzung von 2-Methyl-4(5)-nitroimidazol mit Dimethylsulfat in Ameisensäure bei erhöhter Temperatur besonders vorteilhafte Ergebnisse erhält, wenn man die Umsetzung im Druckbereich von 50—500 mbar durchführt.

Nach dem neuen Verfahren wird die Umsetzung von 2-Methyl-4(5)-nitroimidazol mit Dimethylsulfat in Ameisensäure bei erhöhter Temperatur und bei Drücken von 50 bis 500 mbar, insbesondere 150 bis 250 mbar, durchgeführt. Die Umsetzung wird kontinuierlich in einem Rührkessel einer Rührkesselkaskade oder Bodenkolonne oder diskontinuierlich in einem Rührkessel vorgenommen. Man arbeitet vorteilhaft bei der Siedetemperatur des Umsetzungsgemisches, die sich aufgrund des Druckes und der Konzentration der in der Ameisensäure gelösten Stoffe einstellt und die etwa bei 50 bis 75°C liegt.

Die Ausgangsstoffe 2-Methyl-4-(5)-nitroimidazol und Dimethylsulfat werden in stöchiometrischen Mengen oder mit einem Überschuß an Dimethylsulfat, vorzugsweise in einem Molverhältnis von 1 bis 2 Mol Dimethylsulfat je Mol 2-Methyl-4(5)-nitroimidazol umgesetzt. An Ameisensäure nimmt man zweckmäßig die 1,0- bis 4,0fache Gewichtsmenge, insbesondere die 1,5- bis 2,5fache Gewichtsmenge, bezogen auf das 2-Methyl-4(5)-nitroimidazol.

Im Vergleich zu den bekannten Verfahren erhält man nach dem neuen Verfahren 1,2-Dimethyl-5-nitroimidazol auf besonders wirtschaftlichem Wege und mit besserer Ausbeute und Reinheit. Es können somit umständliche und teuere Reinigungsoperationen minimiert werden. Da der Dimethylsulfat-Umsatz, bezogen auf die Bildung des unerwünschten Nebenproduktes Methylformiat, der bei der Umsetzung unter Normaldruck über 50% beträgt, bei der erfindungsgemäßen Umsetzung unter einem Druck von 180 mbar auf etwa 5% absinkt, erweist sich das Verfahren nach dieser Erfindung als besonders vorteilhaft. Schließlich erhöht das neue Verfahren auch die Betriebssicherheit, da bei einem eventuell auftretenden Leck des Reaktionsgefäßes zunächst Luft in die Anlage eindringen, aber kein hochgiftiges Dimethylsulfat austreten kann.

1,2-Dimethyl-4(5)-nitroimidazol wird z. B. auf dem humanmedizinischen und veterinärmedizinischen Sektor gegen Protozoenerkrankungen verwendet. Es ist außerdem als Zwischenprodukt für die Herstellung von Farbstoffen, Textilhilfsmittel und Insektiziden geeignet.

### Beispiel 1

580 kg trockenes Menidazol mit einem Gehalt an reiner Substanz von 95% und 800 l 85%ige wäßrige Ameisensäure werden in einen Rührbehälter eingefüllt. Daraufhin wird auf 200 mbar evakuiert, der Rührer eingeschaltet, und das Reaktionsgemisch mit 15 bar-Dampf auf ca. 54°C aufgeheizt, so daß die Ameisensäure ein wenig am Rückfluß kocht. Nun pumpt man über einen Zeitraum von 2 Stunden 408 l Dimethylsulfat zu und hält das Gemisch 15 Minuten bei gleichem Vakuum am Sieden. Dann wird die Ameisensäure im Vakuum abdestilliert. Der verbleibende Rückstand wird in 600 l Wasser gelöst und mit Ammoniakwasser auf einen PH-Wert von 1,6 bis 1,8 abgestumpft. Das nicht umgesetzte 2-Methyl-4(5)-nitroimidazol fällt aus und wird trocken geschleudert. Das Gemisch wird anschließend mit Ammoniakwasser auf einen pH-Wert von 9 bis 10 gebracht. Das ausgefallene Produkt (1,2-Dimethyl-5-nitroimidazol) kann nun abgetrennt werden. Man erhält 385 kg 1,2-Dimethy-5-nitroimidazol (Dimetridazol) und 165 kg 2-Methyl-4(5)-nitroimidazol. Somit betragen die Selektivität 90% der Umsatz 70% und die Ausbeute (= Selektivität × Umsatz) 63%.

### Beispiel 2

In einen kontinuierlich betriebenen Rührkessel werden unter 180 mbar Druck pro Stunde 177 g 2-Methyl-4-(5)-nitroimidazol als Maische in 323 g 85% wäßrige Ameisensäure und 180 g Dimethylsulfat eingeleitet. Der Rührkessel wird so beheizt, daß sich eine Reaktionstemperatur von ca. 72°C einstellt. Die Aufarbeitung wird wie im Beispiel 1 beschrieben durchgeführt. Man erhält Dimetridazol mit folgendem Ergebnis: Umsatz 70%, Selektivität 92% und Ausbeute 64,4%.

### Beispiel 3

In eine kontinuierlich betriebene Glockenbodenkolonne (10 Böden, 200 ml Volumen) werden unter 180 mbar Druck auf den obersten Boden pro Stunde 2150 g einer Maische, die aus 761,1 g 2-Methyl-4(5)-nitroimidazol und 1388,9 g 85% wäßrige Ameisensäure besteht, und 750 g Dimethylsulfat eingeleitet. Die Temperatur beträgt im Sumpf ca. 85°C und auf den Böden zwischen 45 und 65°C. Der Glockenbodenkolonne aufgesetzt ist ein Kondensator, in dem das gesamte Destillat mit Ausnahme des angefallenen Methylformiats— also im wesentlichen Ameisensäure und Wasser — kondensiert und in die Kolonne zurückläuft. Der Reaktionsaustrag wird am Sumpf abgezogen. Die Aufarbeitung wird wie in Beispiel 1 beschrieben durchgeführt. Man erhält Dimetridazol mit folgendem Ergebnis: Umsatz 68%, Selektivität: 93% und Ausbeute: 63,2%.

Vergleichsbeispiel

(Beispiel 1 der DE-OS 2 414 280)

600 Teile Ameisensäure (85%ig), 225 Teile 2-Methyl-4(5)-nitroimidazol und 250 Teile Dimethylsulfat werden in einem Rührkeseel 4 Stunden unter Rückfluß erhitzt (80°C). Danach wird die Ameisensäure im Vakuum abdestilliert. Der verbleibende Rückstand wird in 400 Teilen Wasser gelöst, mit Ammoniakwasser auf einen pH-Wert von 1,8 abgestumpft und auf 0 bis +5°C gekühlt. Das nicht umgesetzte 2-Methyl-5-nitroimidazol fällt aus und wird abgeschleudert. Das Gemisch wird dann mit Ammoniakwasser auf einen pH-Wert von 10 eingestellt und bei 10°C 1,2-Dimethyl-5-nitroimidazol abgetrennt. Man erhält 75 Teile 2-Methyl-5-nitroimidazol und 145 Teile 1,2-Dimethyl-5-nitroimidazol vom Fp. 137 bis 140°C. Dies entspricht 66,6% Umsatz, 87% Selektivität und 58% Ausbeute.

**Patentansprüche**

1. Verfahren zur Herstellung von 1,2-Dimethyl-5-nitroimidazol durch Umsetzung von 2-Methyl-4(5)-nitroimidazol mit Dimethylsulfat in Ameisensäure bei erhöhter Temperatur, dadurch gekennzeichnet, daß man die Umsetzung im Druckbereich von 50 bis 500 mbar durchführt.

2. Verfahren nach Anspruch 1, dadurch gekennzeichnet, daß man die Umsetzung im Druckbereich von 100 bis 250 mbar durchführt.

3. Verfahren nach Anspruch 1, dadurch gekennzeichnet, daß man die Umsetzung bei der entsprechenden Siedetemperatur des Umsetzungsgemisches durchführt.

**Claims**

1. A process for the preparation of 1,2-dimethyl-5-nitroimidazole by reacting 2-methyl-4(5)-nitroimidazole with dimethyl sulphate in formic acid at elevated temperature, wherein the reaction is carried out at a pressure of from 50 to 500 mb.

2. A process as claimed in claim 1, wherein the reaction is carried out at a pressure of from 100 to 250 mb.

3. A process as claimed in claim 1, wherein the reaction is carried out at the particular boiling temperature of the reaction mixture concerned.

**Revendications**

1. Procédé pour la préparation de 1,2-diméthyl-5-nitroimidazole par réaction de 2-méthyl-4(5)-nitroimidazole avec les sulfate de diméthyle dans l'acide formique à température élevée, caractérisé en ce qu'on mène la réaction dans la gamme de pression comprise entre 50 et 500 mbar.

2. Procédé selon la revendication 1, caractérisé en ce qu'on mène la réaction dans la gamme de pression comprise entre 100 et 250 mbar.

3. Procédé selon la revendication 1, caractérisé en ce qu'on mène la réaction à la température correspondante d'ébullition du mélange réactionnel.